# EUROPEAN PATENT APPLICATION

(11) **EP 4 814 473 A1**
(43) Date of publication of application: **30.09.2026**
(21) Application number: 25167065.9
(22) Date of filing: 28.03.2025
(51) Int. Cl.: G01N 15/1429, B01L 3/00, C12M 1/00, C12M 3/06, G01N 15/1433, G06V 10/82

(54) **METHOD FOR EVALUATING NANOPARTICLE TRANSPORT ACROSS CELLULAR BARRIERS USING ARTIFICIAL INTELLIGENCE/MACHINE LEARNING (AI/ML) ANALYSIS**

(71) Applicant: Biodevice Systems s.r.o., 10100 Prague (CZ)
(72) Inventor: Goranov, Vitaly, 19000 Prague (CZ)

(57) **Abstract**

This patent application discloses a novel method for evaluating nanoparticle transport across cellular barriers using an integrated microfluidic system combined with artificial intelligence/machine learning (AI/ML) analysis. The approach employs a multi-compartment microfluidic chamber where cell barriers are cultured on porous membranes, enabling real-time assessment of nanoparticle uptake, retention, transport efficiency, and cell viability. Standard light microscopy visualizes label-free nanoparticles within cell organelles, while AI/ML-driven image analysis classifies uptake patterns, quantifies efficiency/rate of nanoparticle transport, and distinguishes between passive and facilitated transport mechanisms. Compared to conventional techniques, this high-throughput, label-free method offers enhanced accuracy and predictive insights into nanoparticle behavior in biological systems, with applications in drug delivery, toxicology, and diagnostics.

## Description

### Field of the invention.

The present invention relates to methods for evaluating the efficiency of nanoparticle transport across cellular barriers. More specifically, this invention integrates a microfluidic chamber with cell barriers, standard microscopy with microscopic image acquisition, and AI/ ML-based analysis of the resulting images for a comprehensive, quantitative assessment of nanoparticle uptake, retention, transport efficiency, and cell viability.

This invention is particularly relevant in the biomedical field, where understanding nanoparticle interactions with cellular barriers is critical for applications in drug delivery, toxicology, and diagnostics. The invention does not aim to reproduce in detail the multilayer cellular barrier as a complete physiological model; however, it enables screening studies of the permeability of specific cell barriers to identify nanoparticles with optimal penetration ability and minimal impact on the viability of barrier cells.

Although conventional techniques are valuable, they often face limitations such as low throughput and challenges in interpreting complex experimental results. The invention provides an innovative approach to assessing nanoparticle uptake, retention, and transport across various cellular barriers, addressing key challenges in drug delivery, toxicology, and biomedical applications.

### Background of the invention.

Nanoparticle interactions with cells are critical for applications in drug delivery, toxicology, and diagnostics [1, 3]. Tracking nanoparticles across cellular barriers, such as endothelial, intestinal, tumor microenvironment, or blood-brain barrier (BBB) models, is essential for understanding their behavior in complex biological environments [4, 35]. Conventional techniques such as fluorescence microscopy and inductively coupled plasma atomic emission spectroscopy (ICP-AES) provide valuable data, but are usually limited by either low throughput, high cost or the inability to correctly interpret experimental results and obtain reliable data of nanoparticle transport across cell barriers [10, 26]. For instance, fluorescence microscopy often requires labeling of nanoparticles, which can alter their behavior and limit the accuracy of the results [1]. Similarly, ICP-AES, while highly sensitive, does not provide spatial resolution or morphological insights into nanoparticle uptake and retention within cells [10].

One of the most common problems with new nanoparticles being developed as drug carriers is their ability to cross biological barriers, e.g. penetration of the gastrointestinal tract, lungs, BBB, etc. This ability is an important prerequisite for a substance to be transported to the site of its potential pharmacological action [3, 6].

Most standard cell models for assessing the permeability of biological barriers to nano-agents are based on a microwell plate format, where cell lines are grown on filter inserts. [17, 28]. For the permeation test, these cell barriers are exposed to the nanoparticles and the flux of these nanoparticles through the cell layer is measured using chemical, optical (fluorescent) etc. These methods usually complex, long-time and expensive [3]. Many specialized labs offer contract services for these studies if the necessary facilities are unavailable in-house. However, results from such permeation experiments often show significant variation and lack precision, with high variability commonly observed. [3, 17]. Often, the permeability of cell barriers to certain nanoparticles, involving both passive and active transport, cannot be assessed using some non-cell-based models such as the PAMPA (Parallel Artificial Membrane Permeability Assay) or the PVPA (Phospholipid Vesicle-based Permeation Assay) model. [3, 26]. Moreover, some conventional cell based models, including widely applied Frantz cell lack reproducibility; the inter-lab variance is so high that data should not be compared even if the same cell line is used [17, 31]. This issue arises due to variability in cell sensitivity and challenges in handling large datasets with standard experimental methods and mathematical tools. [12, 25].

This invention addresses these limitations by combining data analysis obtained using standard microscopy with advanced AI/ML data analysis in a standard microfluidic platform [9, 21]. The granularity of cellular organelles, visualized microscopically by their darkness, serves as an indicator of nanoparticle absorption and retention, with aggregation presumably occurring inside organelles like lysosomes [22, 29]. This approach has been validated using ICP-AES, fluorescent microscopy, and flow cytometry, allowing the detection of nanoparticles without labeling, ensuring results accurately reflect nanoparticle behavior in biological systems [10, 16, 39]. Additionally, the use of AI/ML algorithms enables the processing of large datasets, providing quantitative and predictive insights into nanoparticle transport efficiency, uptake patterns, and cytotoxic thresholds [5, 12, 22, 40]. The integration of these technologies provides a robust and comprehensive approach to evaluating nanoparticle transport across cellular barriers, enabling the optimization of nanoparticle design for improved therapeutic outcomes and diagnostic accuracy [18, 21, 38].

### Summary of the invention.

The aim of the invention is to overcome the lack of robust, cheap and efficient methods for measuring efficiency of nanoparticle transport across biological barriers suitable for standard cell laboratories. In one aspect, the invention provides a microfluidic chamber based on a standard cross-flow cell chip with a barrier assembly for evaluating nanoparticle transport across a cell system immobilized on a porous membrane [30].

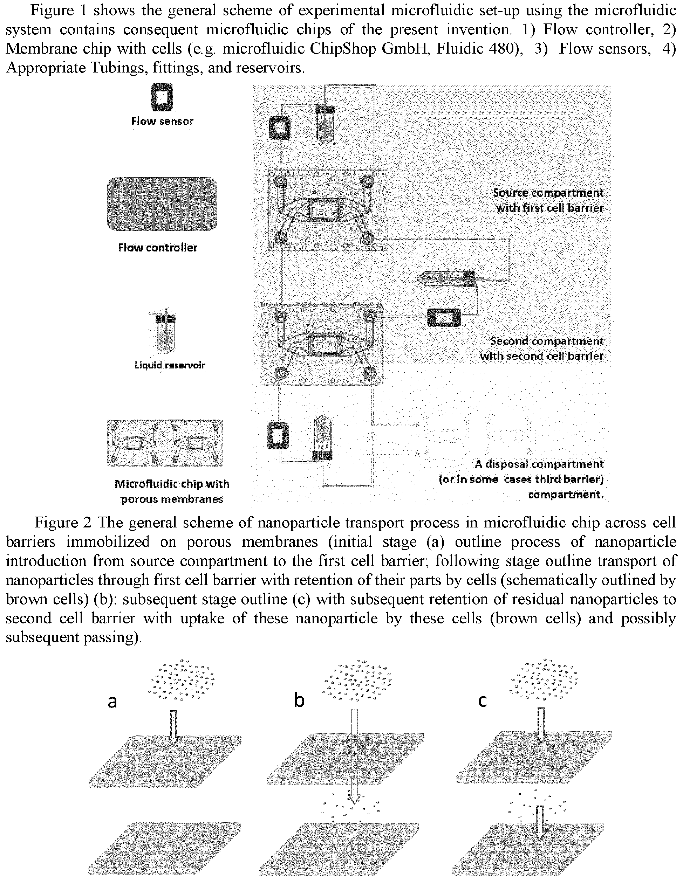

The barrier assembly comprises (fig. 1):
1. A source compartment for introducing a solution containing nanoparticles.
2. A first barrier composed of a first polymeric porous membrane with cells.
3. An intermediate compartment for collecting nanoparticles that have crossed the first barrier, separating the source and intermediate compartments.
4. A second barrier based on a polymeric porous membrane with cells.
5. A disposal (or second intermediate) compartment for collecting nanoparticles that pass through the intermediate compartment(s).
6. Optionally, third barrier based on a polymeric porous membrane with cells.

The barrier assembly mimics cellular barriers found in vivo, such as endothelial, intestinal, and glial cell monolayers [2, 17, 28]. The microfluidic chamber enables long-term exposure of nanoparticles to cell monolayers (or more complex cellular 3D systems), facilitating the study of nanoparticle uptake, retention, and transport efficiency [21, 38]. The system leverages standard microscopy for label-free visualization of nanoparticles accumulated in cell organelles [23]. Following image analysis, using models such as ResAt-UNet [7, 8], provides quantitative data on nanoparticle uptake, retention, and transport efficiency. Cellular darkening, observed via standard microscopy, serves as a visual indicator of nanoparticle uptake and retention within intracellular organelles (primarily lysosomes), with this darkening effect directly correlating with increased cell granularity as measured by image analysis algorithms. [22, 29].

Data from microscopy is processed using AI/ML algorithms, including regression models, to classify uptake patterns, predict transport rates, and assess viability levels. The system achieves high accuracy in data analysis, exemplified by metrics such as an Intersection over Union (IoU) of 0.85, precision of 93.2%, and recall of 86.9%. This integrated approach facilitates the evaluation of nanoparticle transport across diverse cell types and nanoparticle features, enabling optimization for improved drug delivery, diagnostic agents, and toxicological studies. [4, 27, 35].

The system's design includes a microfluidic chamber with multiple chambers and porous membranes for culturing sequential cell monolayers. The porous membranes should have an optimized pore size of approximately 0.4 - 0.6µm, which allows for natural nanoparticles transport across cell barriers retaining the integrity of the cell monolayers [21, 28, 38]. The sequential flow design ensures that nanoparticles pass through each monolayer and enter the next compartment, mimicking the cellular barriers inherent to specific biological system [17, 28]. The microscopy includes standard light microscopy for label-free visualization of nanoparticles accumulated in cell organelles [23]. Automated or manual imaging processes record and store an average of 50 microscopy fields every 6-12 hours (up to 72 hours preferably). This extensive dataset ensures high segmentation accuracy using neural network models like ResAt-UNet, pre-trained with customized, manually labeled images or benchmark datasets. [8, 22]. The system's AI/ML analysis pipeline integrates data from microscopy, using algorithms such as convolution neural network (CNN) for image segmentation, classification of uptake patterns, and regression models for predicting nanoparticle transport rates [12, 29].

Compared to traditional methods like Franz cell chambers, ICP-AES, and fluorescent microscopy, the invention offers advantages, including:
1. Applicability to a wider range of samples, particularly adherent cell monolayers.
2. Capability to process large datasets necessary for data robustness.
3. Evaluation of cell viability.
4. Ease of implementation.
5. Cost-effectiveness.

The biological barrier system according to the present invention may be used in different ways. Thus, an aspect of the invention relates to the use of a barrier assembly, including: (1) the methodology according to the present invention as a tool for evaluating the efficiency of nanoparticle transport across biological barriers, and/or (2) determining the stability/viability of the investigated biological barrier for investigated nanoparticles at different concentrations [25, 35].

The invention stands out from other methods for measuring of nanoparticles transport efficiency in defined biological environment, by its simplicity and precision in terms of applications for studying different cell types [21, 28, 36]. Furthermore, this approach is fully compatible with the development and proliferation of living cells and allows to address the dynamic variability of living cell systems using AI/ML analysis [12, 14, 15, 22].

Beyond the information on the transport of nanoparticles in cell systems, the versatility and simplicity of implementation of the method promises an application in biomedical industrial research contexts [9, 38]. For example, the invention could serve for standardization and rapid, accurate classification of nanoparticle efficiency. It can establish rapid assays to screen the transport rate, which depends not only on the nature of the investigated nanoparticles but also on the permeability of cell systems. These systems can be treated with various active factors to mimic barrier conditions in normal and pathological states. This allows for choosing optimal manufacturing signatures useful in diagnosing unhealthy tissues in the field of health and evaluating the effect of different biological factors on the permeability of cell barriers [35, 38].

The application of a magnetic field during the testing of nanoparticles with a magneto-responsive core significantly enhances the evaluation process within the microfluidic chamber system. This approach enables researchers to observe directed nanoparticle transport under controlled magnetic conditions, providing deeper insights into their behavior across biological barriers. The magnetic field manipulation not only facilitates precise targeting studies but also allows for partial predictions regarding the effectiveness of improved localized drug delivery in vivo. By controlling nanoparticle movement through external magnetic forces, researchers can optimize their targeting capabilities using the sequential barrier system, ensuring that therapeutic agents would be delivered precisely to the desired site within complex biological systems. The integration of magnetic field control within the standardized microfluidic chamber setup adds another dimension to transport analysis, offering quantifiable data on magnetically-guided nanoparticle distribution and retention in specific cell monolayers, thereby advancing the development of more effective treatment strategies for targeted therapy and personalized medicine [34, 35].

### Detailed description of the invention.

The subject of the invention is an in vitro method for characterizing at least one integral parameter of the transport rate of at least one investigated nanoparticle across at least one cellular barrier. This method comprises:
a) Introducing a suspended nanoparticle sample (using a biocompatible solution) of varying concentrations into the source compartment of a microfluidic chamber. This allows for nanoparticle uptake or transport across a cell monolayer immobilized on a porous membrane, with subsequent transfer into an intermediate compartment (fig.2). It also allows for following nanoparticle uptake and retention by cells immobilized on a second polymeric porous membrane, or optionally by a second or third cell barrier immobilized on polymeric porous membranes. [21, 28];
b) Periodically recording cell monolayer microphotographs in digital form using standard or automatic microscopy for at least 50 fields of vision; c) Analyzing transport efficiency and/or cell viability using a pre-trained ResAt-UNet (or similar) neural network [7, 8, 22].

Preferably, the core of the investigated nanoparticles includes super para magnetic nanoparticles (SPIONs) or other particles that contain magnetically responsive components (MNPs). The nanoparticles with this core demonstrated the best recognition under standard microscopy and following analysis with AI/ML [13, 20, 23, 34]. The features of such nanoparticle shape, size, and shell primarily define their transport properties across biological barriers and the viability of cells comprising these barriers (excluding the case of rapidly biodegradable shells) [1, 4, 19].

The method according to the invention is compatible with all kinds of nano-and micro-particles (with and without magnetically-responsive core) [3, 6]. There are a large number commercially available, with a range of adjustable surface chemistry [19, 33]. The inventors tested and validated the method according to the invention with about ten types of nano-and micro-particles of different brands, without any sign of defect or limit of the approach [21, 38].

It is important to note that the application of this method is limited to subtoxic concentrations of nanoparticles [24, 26]. While these concentrations may reduce cell viability, they do not cause significant destruction of cellular components within the studied monolayer, nor do they compromise the integral integrity of the porous membrane, which could otherwise distort nanoparticle transport metrics [25, 35]. By imposing this limitation, we acknowledge that nanoparticles with high toxic potential are unlikely candidates as carriers for active agents and drugs at the level of barrier tissues [3, 26].

On the other hand, by carefully adjusting the concentration of the administered nanoparticles, a balance can be achieved that minimizes damaging effects while ensuring an adequate quantity of particles for visualization within the cells for subsequent analysis [4, 25]. This approach allows for the effective use of proposed methodological approach while maintaining cellular integrity and providing reliable data for AI/ML analysis [12, 22].

### System/Tool Overview

### 1. Microfluidic Chamber with cell barrier(s)

o **Design:** A standard cell cross-flow microfluidic chamber(s) with porous membrane compartment(s) with cell monolayers. The chamber is necessary to mimic the complex cellular barriers found in biological systems. The pore size of the membranes is optimized at 0.1 - 0.4 µm to allow controlled diffusion of nanoparticles while retaining the integrity of the cell monolayers.
∘ **Cell barriers:** Endothelial, mesenchymal stem cells (MSCs), fibroblasts, glial cells, intestinal etc., are cultured on porous membranes within one or more connected microfluidic chambers [17, 32]. The integrity of the cell monolayers can be verified via TEER value > 120 Ω*cm² (can be defined using any available equipment) [28]. Any standard approach to free pore sealing can be used in order to improve parameters of porous/cell barrier integrity and avoiding of nanoparticle seeping through the free pore space in case of sub-confluent monolayer with TEER control [21]. In the last case any standard biocompatible dyes can be applied to test nanoparticle seeping through the free pore space [39].
∘ **Flow Parameters:** The sequential flow design ensures that nanoparticles pass through each monolayer and enter the next compartment. This design allows for the long-term exposure of nanoparticles to cell monolayers, enabling the study of nanoparticle uptake, retention, and transport efficiency across these barriers. This configuration enables circulating of homogenous suspension of nanoparticles in different compartments and efficient exposure of nanoparticles to the cell monolayers, providing a robust platform for studying nanoparticle uptake, retention, and transport efficiency across these biological barriers up to 72h. By optimizing flow parameters such as flow rate (50-200 µL/min), pressure (0.1-0.5 kPa), maintaining of laminar flow and other regimes a temperature of 37°C with 5% CO₂ concentration (the last can be supported by external CO₂-incubator), the microfluidic system can be fine-tuned to replicate physiological conditions, thereby enhancing the relevance and applicability of experimental findings [17, 38].

### 2. Microscopy

o **Imaging Technique:** Standard light microscopy for label-free visualization of nanoparticles accumulated in cell organelles [23]. Can be used standard microscopy tools with manually fulfilled investigation of any Automatic cell imaging system with appropriate recognition for recording of obtained images that allows for the detection of nanoparticles without the need for labeling [23]. According to our observations, reliable recognition of cell granularity associated with nanoparticle accumulation in cell organelles accompanied by their darkening and corresponding increase of cell granularity, as well as cell shapes, is achievable using images captured at 40x magnification [22]. At least 50 microscopy fields should be recorded [25].
∘ **Morphological Analysis for evaluation of cell viability:** According to our experience, the most representative parameter that can be used alone or in complex with other parameter like nuclear/cytoplasm ratio or other morphometric parameters) is elongation index (the parameter successfully analyzed by AI in micrographs) [25]. Cells with high uptake that can induce sub-lethal events (inhibit proliferation, induce cell apoptosis after 2-3 days of incubation) usually exhibited elongation indices >1.8 (or have abnormally low sizes like apoptotic bodies) [24]. The morphological analysis provides quantitative data on the uptake and retention of nanoparticles within cells, enabling the classification of uptake patterns and the prediction of transport rates [22].
∘ **Granularity Detection for evaluation of nanoparticle uptake:** The microscopically detected granularity in cells (associated with darkening of cellular organelles) serves as an indicator of the uptake and retention of nanoparticles, with their aggregation within the intracellular organelles (lysosomes presumably) [29]. The granularity detection provides insights into the distribution and accumulation of nanoparticles within cells, enabling the evaluation of their transport efficiency across cellular barriers [22]. AI/ML analysis successfully provides reliable results in the dynamic cell incubation with nanoparticles (validated in for the described examples using ICP-AES, fluorescent microscopy, and flow cytometry techniques). In contrast, standard manually performed analysis of images (including high-throughput microscopy) with subsequent statistical analysis is often complex and may not provide reliable results [23].

### 3. Mathematical analysis

AI/ML models evaluate and compare data from microscopy for final decision about nanoparticle transport efficiency across the cell barrier. The data integration enables the processing of large datasets, providing quantitative and predictive insights into nanoparticle transport efficiency, uptake patterns, and cell viability.

### The AI/ML analysis pipeline comprises the following steps:

### Step 1: Preprocessing of Cell Images:

▪ Normalizing the brightness and contrast of microscopy images (micrographs); applying noise reduction techniques, such as Gaussian blur or median filtering, to enhance image quality [40].

### Step 2: Image Segmentation:

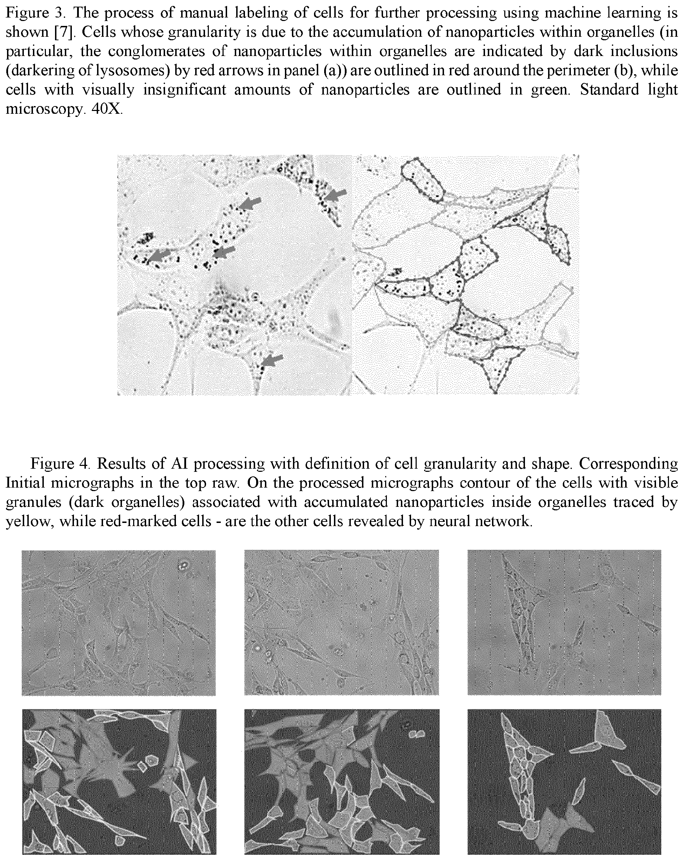

▪ Using a pre-trained CNN, such as ResAt-UNet (alternatively, other CNNs with similar parameters can be used), to segment cell organelles with increased darkness and accordingly recognize cell granularity in the images [7, 8, 29]. In particular, the ResAt-UNet neural network allows us to achieve high segmentation accuracy. The networks are trained with adapted, manually labeled images (Fig.3). These algorithms achieve robust data analysis with performance metrics (e.g. in our studies with an IoU of 0.85, a precision of 93.2% and a recall of 86.9). Segmentation identifies regions corresponding to aggregates of nanoparticles within organelles (Fig. 4), such as lysosomes [37]. Features such as granularity intensity, granularity square (overall and relative per cell or cell' square with granularity above the threshold), cell shape and elongation index are extracted from the segmented images [22]. These features serve as input for subsequent classification and regression analyzes and allow a quantitative assessment of nanoparticle uptake and retention.
**▪ Step 3: Classification of Uptake Patterns (optional, mostly improves the analysis using Long Short-Term Memory neural network (LSTM):**
   This step analyzes nanoparticle uptake patterns within cells through granularity detection and pattern classification. Granularity detected identifying nanoparticle aggregates in organelles using pixel intensity thresholds (0-255 scale). Aggregates are typically detected when intensity exceeds 2.5× background intensity. Maps granularity trends to uptake rates and monitors cell shape changes to identify potential cytotoxic thresholds during dynamic analysis.

Pattern Classification: Employs k-means clustering to objectively categorize uptake patterns without requiring labeled training data [12]. For example:
1. Implementation uses k-means with k=3 and Euclidean distance metrics
2. Classification based on key features including:
   ▪ Granularity intensity (identified granules per cell)
   ▪ Elongation index (cell length/width ratio)
   ▪ Granularity density (granules per cell or per µm²)

This classification enhances downstream analysis by grouping similar uptake behaviors based on granularity distribution, intensity, and localization patterns.

### Step 4: Comparative Analysis:

▪ (1) Using multiple linear regression model to predict nanoparticle transport rates based on the classified uptake patterns and time-dependent changes in the images [12]. Comparing the results obtained using evaluation of images of cells between a first polymeric porous membrane carrying a cellular barrier layer and a second polymeric porous membrane (and second and third if it necessary for different cell kinds) carrying a second cellular barrier layer to obtain values for nanoparticle transport efficiency (%) [21]; Example of evaluation: Transport Efficiency = β₀ + β₁(Granularity_Barrier1) + β₂(Granularity_Barrier2) +...+ ε
   Where β₀-βx are regression coefficients and ε is the error term [12].
▪ (2) Simultaneously monitoring of relative cell viability evaluated using analysis of such morphometric parameter as cell elongation (Cells with high uptake that can induce sub-lethal events (inhibition of proliferation, cell apoptosis exhibited elongation indices >1.8) [25]. Whereas if percent of cells with elongation indices >1.8 exceed 25% the reliability of evaluation of transport efficiency decrease lower threshold of p>0.1 [25].

### Step 5 (optional): Distinguishing of of base transport pathways using LSTM

Predominantly Passive Transport: Nanoparticles cross barriers via simple diffusion driven by concentration gradients or Brownian motion, without cellular energy expenditure [3]. Key Features:
▪ Linear or gradual increase in nanoparticle accumulation (granularity intensity) over time.
▪ Minimal cell shape changes (elongation indices <1.8).
▪ Size-dependent efficiency (smaller nanoparticles, e.g., 15 nm SPIONs, diffuse more readily) [4].

Predominantly facilitated transport: Nanoparticles are transported via energy-dependent mechanisms (e.g., receptor-mediated endocytosis, transcytosis) [3]. Key Features:
▪ Non-linear or abrupt spikes in granularity intensity (e.g., rapid lysosomal uptake).
▪ Higher cell stress markers (elongation indices >1.8) due to energy expenditure and decrease of viability.
▪ Size and surface chemistry influence active uptake (e.g., larger nanoparticles like 150 nm MNP s require active processes) [4].

### Step 6: Statistical analysis

▪ Performing statistical analysis using a standard approaches (e.g. ANOVA) to validate the transport efficiency values and assess their significance.

Thus, the integrated analysis pipeline, combining pre-trained neural networks (e.g., ResAt-UNet) with statistical software packages, enables users to rapidly generate reliable metrics for nanoparticle transport efficiency and cell viability without requiring specialized expertise in AI/ML. By automating image segmentation, clustering, and regression analysis, the system simplifies data interpretation, outputting standardized reports that include transport rates (%), viability thresholds, and predictive insights (e.g., optimal nanoparticle size or coating). This user-friendly approach ensures reproducibility across laboratories, as the pre-trained models mitigate variability inherent to manual analysis, while the statistical framework validates significance (p < 0.05) and power (>90%) for robust conclusions. The transport efficiency values provide insights into the optimization of nanoparticle design for improved therapeutic outcomes and diagnostic accuracy [4, 35].

Finally, the biomimetic cell barriers of the present invention exhibit good compatibility with a diverse array of nano- and micro-objects [21, 28]. Comprehensive protocols for maintaining these cells under microfluidic conditions are well-documented and readily accessible for most laboratories [28]. These protocols include the use of specialized culture media, essential supplements, and environmental parameters such as temperature, CO₂ concentration, and medium flow rates [17, 38].

### Scope of the method

The proposed method utilizes a cell chamber to facilitate the transport of nanoparticles through specific microfluidic biochip compounds, followed by the evaluation of microscopy images using machine learning (ML) and artificial intelligence (AI) techniques. This innovative approach enables the exploration of transport efficiency for various nanoparticles across cellular barriers and allows for the assessment of how this transfer impacts cell viability.

In one aspect, the method is applicable to multicellular biological samples, enabling the characterization of at least one type of nanoparticle and one specific cell type in the context of nanoparticle transport. This dual characterization provides valuable insights into the interactions between nanoparticles and cellular environments.

In another aspect, the method allows for the evaluation of cell viability in relation to the transport of specific nanoparticles. This is crucial for understanding the safety and efficacy of nanoparticle-based therapies.

The invention encompasses the application of this method for several key purposes:
1. Characterization of Transport Efficiency: To assess the ability of specific types of nanoparticles to effectively traverse various cell barriers, providing insights into their potential for targeted drug delivery.
2. Evaluation of Cell Barrier Properties: To determine the capacity of different cell barriers to be efficiently crossed by a range of nanoparticle types, which is essential for optimizing therapeutic strategies.
3. Impact of Therapeutics on Transport Efficiency: To investigate how drugs or medications influence the transport efficiency of different nanoparticles across various cell barriers, thereby enhancing the understanding of drug-nanoparticle interactions.
4. Screening for Cellular Damage and Barrier Integrity: To identify and quantify cellular damage and alterations in the permeability of cell barriers under the influence of various nanoparticles at different concentrations. This screening process is vital for assessing the safety profile of nanoparticle formulations.

This method combines standard imaging and tissue culture techniques with ML/AI analysis to create a comprehensive platform for investigating nanoparticle transport dynamics, advancing the development of safer and more effective nanoparticle-based therapeutic solutions [11].

The cellular barriers can be prepared using well-established processes commonly employed in cell culture methodology, utilizing standard microfluidic systems with cross-flow cell chips. This highly adaptable setup can be readily modified to meet specific research requirements, with customizable microfluidic chambers and barrier cell layers tailored to specific experimental needs.

Barrier features can be adapted to various experimental configurations, such as blood-brain barrier (BBB) models or other, with virtually no limitations on size (diameter) or shape (round, elongated, etc.). Both cell barriers and complete devices can be mass-produced and preserved for extended periods under standard freezing protocols, enabling straightforward shipping to end users while maintaining cellular viability through proper restoration procedures.

Rather than attempting to replicate detailed physiological cellular barriers, this technology serves as an efficient screening platform for evaluating how cell systems and their combinations interact with nanoparticles. This approach specifically identifies candidates with superior transport capabilities while minimizing cytotoxic effects on barrier cells.

An important advantage of this technology is its versatility - not being restricted to any specific test format or commercial platform. It functions effectively in commercially available microfluidic chips and alternative devices, potentially circumventing current size limitations for permeation cell barriers for various nanoobjects. With the ability to produce barriers in virtually any dimension, experimental configurations can be customized for specific applications, including automation and in-line measurement systems.

The AI/ML analysis provides a powerful quantitative assessment of nanoparticle transport across cellular barriers - critical for applications in drug delivery, nanotoxicology and diagnostics. The system provides quantitative and predictive insights into transport efficiency, uptake patterns and cytotoxic thresholds, supporting the elaboration of optimized nanoparticle design for improved therapeutic outcomes and diagnostic precision.

### Examples

### Example 1. Simulation Workflow to Analyze Nanoparticle Transport across Sequential Cellular Barriers Using AI/ML

### (a) Acquiring Time-Lapse Microscopy Images

The workflow begins with acquiring time-lapse microscopy images of sequential cellular barriers in a microfluidic chamber. The chamber comprises two polymeric porous membranes (pore size: 0.4 µm) supporting endothelial cell monolayers, with integrity verified via TEER >150 Ω·cm². Images are captured at 40x magnification every 6 hours for up to 72 hours, ensuring 50 fields of view per time point to statistically robust datasets.

### (b) Preprocessing the Microscopy Images.

Raw images undergo preprocessing to enhance contrast and reduce noise. Brightness normalization is achieved via histogram equalization, while noise reduction employs Gaussian blur (kernel size: 3x3) and median filtering (window size: 5x5).

### (c) Analyzing the Microscopy Images.

A pretrained ResAt-UNet model (IoU: 0.85, precision: 93.2%, recall: 86.9%) segments cellular regions and organelles, leveraging a custom dataset of 5,000 manually labeled images augmented with rotations and flips. This step ensures granularity detection accuracy, critical for quantifying nanoparticle accumulation in cell lysosomes. The same CNN where used for morphological analysis via calculates elongation indices (cell length/width), where indices >1.8 indicate sub-lethal stress (e.g., inhibited proliferation, early apoptosis), correlating with viability drops e.g. for Bare SPIONs (in Tables 2 and 4).

### (d) Classification of data.

The k-means clustering algorithm (k=3, Euclidean distance metric) groups cells into three biologically relevant clusters based on granularity (nanoparticle aggregates per cell), elongation index (cell length/width ratio), and inferred viability thresholds. Clustering parameters are defined by three key parameters: (1) granularity count (thresholded at >3 granules/cell for "high uptake" and >10 granules/cell for "hyper-accumulation"), (2) elongation index (viability threshold at <1.8 for preserved morphology), and (3) spatial distribution of granules within organelles (e.g. lysosomal).

The clusters are:
Cluster 1 (High Uptake, Viable): Cells with 3-10 granules/cell and elongation <1.8, indicating efficient nanoparticle internalization without cytotoxicity.
Cluster 2 (Hyper-Accumulation, Stressed): Cells with >10 granules/cell and elongation >1.8, linked to lysosomal overload, reduced viability, and impaired barrier integrity.
Cluster 3 (Low Uptake, Baseline): Cells with <3 granules/cell and elongation <1.8, representing minimal nanoparticle interaction and intact barrier function.

For example, in endothelial monolayers exposed to 30 nm Polylactide-co-glycolide coated magnetic nanoparticles (PLGA- MNPs) in concentration 50 µg/mL, clustering revealed 62.4% Cluster 1, 18.9% Cluster 2, and 18.7% Cluster 3 cells. This model achieved R² = 0.91 for endothelial cells, outperforming granularity-only models (R² = 0.78) by accounting for viability-linked heterogeneity. Cluster-specific trends align with Table 1 data, where PLGA-coated SPIONs exhibited 68.9% transport efficiency, driven by dominance of Cluster 1 cells. Conversely, bare SPIONs at 50 µg/mL showed reduced efficiency (28.5%) due to higher % Cluster 2 cells (41.2%), validating the model's predictive capability. The cluster-specific temporal trends (e.g., increasing % Cluster 2 cells over 72 hours) serve as critical input features for the Long Short-Term Memory (LSTM) network, improving its ability to model time-dependent transport mechanisms.

### (e) Comparing analysis for the relative transport efficiency evaluation

Multiple linear regression analysis was employed to compare nanoparticle accumulation between sequential barriers. The model evaluate relative transport efficiency based on granularity differences between barriers. Example of evaluation: Transport Efficiency = β₀ + β₁(Granularity_Barrier1) + β₂(Granularity_Barrier2) +...+ ε

Where β₀-βx are regression coefficients and ε is the error term.

For example, the differences in granularity between the first and second barriers result in a transport efficiency of 65.4 ± 2.3 % for 30 nm SPIONs in endothelial barriers. For the same SPIONs, the transport efficiency, which is enhanced by the influence of 1T magnetic fields, is 73.2 ± 8.1 % (Table 3).

### (f) Time-Dependent Feature Extraction.

Time-dependent features are extracted from microscopy data to capture dynamic nanoparticle-cell interactions across sequential time points. This includes nanoparticle density per compartment (particles/µm²), which maps spatial distribution trends, and temporal granularity slopes (Δintensity/hour), quantifying the rate of nanoparticle accumulation in organelles such as lysosomes. Concurrently, viability metrics like the percentage of elongated cells (elongation index >1.8) or nuclear/cytoplasmic ratio shifts are tracked to assess stress responses. For instance, a 30 nm SPION at 50 µg/mL exhibits a granularity slope of **+1.2 units/hour** in endothelial cells, reflecting progressive loading. These features are structured into sequential time-series datasets (e.g., 6-hour intervals over 72 hours), enabling the Long Short-Term Memory (LSTM) network to model temporal dependencies between nanoparticle uptake, cellular stress, and barrier integrity.

### (g) Application of Short-Term Memory Network.

A 3-layer LSTM (128 units/layer, dropout: 0.2) processes time-series features (e.g., granularity, elongation) over 72 hours. Training uses Adam optimization (learning rate: 1e-4, batch size: 32) for 100 epochs, achieving 89% accuracy in classifying predominante transport mechanisms (passive vs. facilitated). Gradual granularity slopes (e.g., +0.8 units/hour) paired with stable viability **(<15% elongated cells)** are indicative of **passive diffusion,** characterized by linear accumulation. Conversely, abrupt granularity spikes (e.g., +3.5 units/hour at 48 hours) with viability drops **(>30% elongated cells)** correlate with **facilitated transport** (e.g., receptor-mediated endocytosis), triggering nonlinear predictions. By integrating clustered data-such as the temporal evolution of % Cluster 2 cells (hyper-accumulation)-the LSTM discerns patterns linking cytotoxicity to transport efficiency. For example, a rise in Cluster 2 cells from 18% to 41% over 72 hours predicts barrier dysfunction, correlating with reduced transport efficiency (68.9% → 53.2%). These predictions align with Table 1 data, where 15 nm of slightly-transported SPIONs show previously passive transport (49.2± 5.3 % efficiency), while 30 nm LSDM- nanoparticles cross the barrier by facilitated diffusion (65.4± 5.3 %).

### (h) Validating Results

Polynomial regression was employed to correlate nanoparticle properties with transport efficiency. This approach captures non-linear relationships while maintaining interpretability. A second-order polynomial model was fitted:
Transport Efficiency = β₀ + β₁(Size) + β₂(Charge) + β₃(Size²) + β₄(Charge²) + β₅(Size×Charge) + ε For example, 50 nm particles show inverse correlations between size and transport across endothelial barrier (38.4% at 1 µg/mL vs. 41.1% at 50 µg/mL, (Table 1).

### (i) Outputting Quantified Metrics

Statistical significance was assessed using standard t-tests and ANOVA with post-hoc Tukey's test (p < 0.05 considered significant). Final integral outputs include nanoparticle transport efficiency (%) across barriers (e.g., 68.9% for 30 nm SPIONs in endothelial cells, Table 1), viability thresholds (% survival <85% flags cytotoxicity), and predictive models for optimized designs (e.g., 15-30 nm SPIONs with PEG shells). Power analysis determined that the sample size provided 90% power to detect differences of 0.15 or greater in transport efficiency at α = 0.05, validating the statistical robustness of the findings.

Example 2. Evaluation of Nanoparticle Transport Efficiency (%) Across Cellular Barriers w/o external magnetic field influence (values in tab.1).

Materials/Tools:
1. Microfluidic chamber with cross-flow cell chips contained porous membranes (0.1-0.4 µm pore size).
2. Endothelial, MSCs and glial cells (correspondingly HUVEC; Bone marrow-derived MSCs; U87MG human glioblastoma cells).
3. MNP s of varying sizes (15 - 150 nm): bare and with surface modifications (PEGylated, uncoated, PLGA -coated).
4. Standard cell culture media and supplements (DMEM/F12, 5%FBS).
5. Standard light microscope (40x magnification).
6. AI/ML software with pre-trained ResAt-UNet.
7. CO₂ - incubator.

Procedure:
1. Culture endothelial, MSC and glial cells on porous membranes each type seeded within separate microfluidic chambers on consequent membranes until monolayers are formed (TEER >120 Ω·cm²).
2. Introduce nanoparticle suspensions (1 µg/ml, 10 µg/ml, 50 µg/ml) into the source compartment of the microfluidic chamber.
3. Maintained constant flow rate (100 µL/min) of microfluidic system and placed in CO₂ incubator at 37°C with with 5% CO₂ for up to 72 hours excluding periods of observations with light microscopy.
4. Acquired microscopy images (50 fields of vision) every 6 hours using standard light microscopy and recording in JPEG files.
5. Preprocess images using noise reduction techniques (Gaussian blur, median filtering).

### Data Analysis (Same as Example 1)

Results:
- PLGA-coated SPIONs (30 nm, 50 µg/mL) have the highest transport efficiency (68.9 ± 3.7% in endothelial cells, 58.7 ± 4.2% in MSCs, and 43.5 ± 5.1% in glial cells).
- PEGylated SPIONs (15 nm) demonstrated moderate transport efficiency (45.3 ± 2.8% in endothelial cells at 10 µg/mL), with efficiency increasing proportionally with concentration (54.1 ± 3.3% at 50 µg/mL).
- Transport efficiency follows the pattern: endothelial cells > MSCs > glial cells, with the most significant difference observed for PLGA-coated 30 nm MNP s (68.9% vs. 43.5%).

**Table 1: Nanoparticle Transport Efficiency (%) Across Cellular Barriers (** - is not applicable)**

| MNP Type | Diameter of SPION's core (nm) | Concentration initial (µg/mL) | Nanoparticle Transport Efficiency (%) | | |
|---|---|---|---|---|---|
| | | | Endothelial | MSCs | Glial Cells |
| | 15 | 1 | 49.2 ± 9.8 | 33.5 ± 7.9 | 17.6 ± 5.6 |
| | | 10 | 51.5 ± 1.3 | 35.7 ± 1.4 | 20.3 ± 8.1 |
| | | 50 | 53.3 ± 1.7 | 37.4 ± 4.9 | 19.1 ± 2.5 |
| | 30 | 1 | 65.4 ± 1.3 | 45.3 ± 3.4 | 22.8 ± 6.1 |
| | | 10 | 67.2 ± 3.1 | 46.8 ± 7.8 | 23.9 ± 6.9 |
| | | 50 | 68.9 ± 3.7 | 47.9 ± 1.2 | 24.8 ± 7.3 |
| | 50 | 1 | 38.4 ± 8.3 | 26.3 ± 6.8 | 13.8 ± 4.3 |
| Rapidly Transported (PLGA -coated) | | 10 | 40.0 ± 4.7 | 27.4 ± 7.1 | 14.7 ± 2.6 |
| | | 50 | 41.1 ± 9.0 | 28.5 ± 7.5 | 15.3 ± 4.9 |
| | 100 | 1 | 30.6 ± 8.1 | 21.5 ± 6.6 | 10.8 ± 1.2 |
| | | 10 | 32.9 ± 8.6 | 23.1 ± 7.0 | 11.9 ± 4.6 |
| | | 50 | 34.7 ± 6.1 | 24.6 ± 7.5 | 12.9 ± 5.0 |
| | 150 | 1 | 14.3 ± 4.4 | 9.7 ± 3.2 | 5.1 ± 2.1 |
| | | 10 | 15.2 ± 4.7 | 10.7 ± 3.5 | 5.5 ± 2.4 |
| | | 50 | 16.4 ± 5.1 | 11.8 ± 3.8 | 6.0 ± 2.7 |
| | 15 | 1 | 25.4 ± 6.3 | 16.3 ± 5.2 | 8.2 ± 3.1 |
| | | 10 | 26.9 ± 6.7 | 17.4 ± 5.6 | 8.7 ± 3.4 |
| | | 50 | 28.5 ± 7.1 | ** | ** |
| | 30 | 1 | 48.4 ± 5.3 | 30.3 ± 8.4 | 15.2 ± 5.4 |
| | | 10 | 50.0 ± 4.7 | 31.8 ± 8.8 | 16.4 ± 1.8 |
| | | 50 | ** | ** | 17.5 ± 0.1 |
| | 50 | 1 | 30.4 ± 8.4 | 19.3 ± 6.9 | 9.7 ± 4.2 |
| Bare MNP s | | 10 | 32.0 ± 8.8 | 20.7 ± 7.3 | 10.8 ± 4.6 |
| | | 50 | 33.6 ± 9.3 | ** | ** |
| | 100 | 1 | 15.4 ± 0.5 | 10.3 ± 4.5 | 5.2 ± 2.8 |
| | | 10 | 16.9 ± 5.9 | 11.4 ± 4.8 | 5.7 ± 3.1 |
| | | 50 | ** | ** | ** |
| | 150 | 1 | 10.3 ± 4.2 | 6.8 ± 3.3 | 3.4 ± 2.1 |
| | | 10 | 11.2 ± 1.6 | 7.7 ± 3.6 | 3.9 ± 0.4 |
| | | 50 | 12.5 ± 5.0 | 8.9 ± 3.9 | 4.5 ± 2.7 |
| | 15 | 1 | 17.4 ± 5.5 | 11.3 ± 4.6 | 6.2 ± 3.1 |
| | | 10 | 18.9 ± 5.9 | 12.5 ± 1.0 | 6.7 ± 3.4 |
| | | 50 | 20.5 ± 6.4 | 13.7 ± 5.4 | 7.3 ± 3.7 |
| | 30 | 1 | 32.4 ± 8.4 | 22.3 ± 6.9 | 11.4 ± 5.2 |
| | | 10 | 33.9 ± 8.8 | 23.8 ± 7.3 | 12.5 ± 5.6 |
| | | 50 | 35.6 ± 9.3 | 25.4 ± 7.7 | 13.6 ± 6.0 |
| Slowly Transported (PEGilated) | 50 | 1 | 20.4 ± 6.6 | 14.3 ± 5.8 | 7.4 ± 3.9 |
| | | 10 | 22.0 ± 7.0 | 15.8 ± 6.2 | 8.2 ± 4.3 |
| | | 50 | 23.7 ± 7.5 | 17.5 ± 6.7 | 9.1 ± 4.8 |
| | 100 | 1 | 14.6 ± 5.8 | 10.1 ± 4.7 | 5.2 ± 2.9 |
| | | 10 | 16.5 ± 3.2 | 11.6 ± 5.1 | 5.9 ± 3.2 |
| | | 50 | 18.6 ± 6.7 | 13.3 ± 0.6 | 6.8 ± 3.6 |
| | 150 | 1 | 7.3 ± 3.4 | 4.8 ± 2.8 | 2.6 ± 1.9 |
| | | 10 | 8.2 ± 3.8 | 5.7 ± 3.1 | 3.0 ± 2.3 |

Example 3. Cell Viability (% Survival) after 72-Hour Exposure w/o external magnetic field influence (outlined in tab.2).

### Materials: (Same as Example 2)

Procedure:
1. Same as Example 2, steps 1-6.
2. Morphological Analysis for evaluation of cell viability: According to our experience, the most representative parameter that can be used alone or in complex with other parameter like nuclear/cytoplasm ratio is elongation index (Features successfully analyzed by AI). Cells with high uptake that can induce sub-lethal events (inhibit proliferation, induce cell apoptosis after second day of incubation exhibited elongation indices >1.8. The morphological analysis provides quantitative data on the uptake and retention of nanoparticles within cells, enabling the classification of uptake patterns and the prediction of transport rates.
3. Used AI/ML algorithms to analyze cell morphology (elongation index) from microscopy images.
4. Calculating of the percentage of viable cells based on morphological characteristics using pre-trained ResAt-UNet neural network.

Data Analysis:
1. Evaluated cell viability using AI/ML based on cell elongation.
2. Cells with high uptake that can induce sub-lethal events exhibited elongation indices >1.8.
3. The morphological analysis provides quantitative data on the uptake and retention of nanoparticles within cells, enabling the classification of uptake patterns and the prediction of transport rates.

Results and analytical outcomes:
- PLGA-coated MNP s maintained high cell viability across all concentrations (97.8 ± 1.2% at 10 µg/mL to 94.3 ± 2.1% at 50 µg/mL in endothelial cells).
- Higher concentrations (50 µg/mL) reduced viability compared to lower concentrations (e.g., 93.4% to 86.5% for PLGA-coated 30 nm MNP s in endothelial cells).
- PEGylated MNP s (30 nm) demonstrated dose-dependent cytotoxicity, with viability decreasing from 95.7 ± 1.8% at 1 µg/mL to 84.2 ± 3.5% at 50 µg/mL in MSCs.
- Bare MNP s exhibited significant cytotoxicity directly correlated with initial concentration (70.8 ± 8.9% viability for 15 nm at 50 µg/mL in endothelial cells).
- Glial cells showed higher sensitivity to nanoparticle exposure, with viability decreasing by an additional 7-12% compared to endothelial cells across all nanoparticle types.
- AI analysis revealed that the experimental sets that contains the large amount of cells with elongation indices >1.8 correlated with decrease of cell viability <70%, imitates application of cell barriers with for reliable analysis of Nanoparticle Transport Efficiency. E.g. bare nanoparticles in concentration of 50 µg/mL should be excluded from the experiments for analysis of Nanoparticle Transport Efficiency across MSC and glial cell barriers that is indirectly supported by high dispersion of corresponding values in tab. 1.

**Table 2: Cell Viability (% Survival) After 72-Hour Exposure**

| MNP Type | Diameter of MNP 's core (nm) | Concentration in source compartment (µg/mL) | Cell viability (%) | | |
|---|---|---|---|---|---|
| | | | Endothelial Cells | MSCs | Glial Cells |
| | 15 | 1 | 97.2 ± 3.4 | 96.1 ± 3.8 | 94.8 ± 4.3 |
| | | 10 | 95.7 ± 4.2 | 94.3 ± 4.7 | 92.5 ± 5.1 |
| | | 50 | 93.4 ± 5.3 | 91.6 ± 5.7 | 89.1 ± 6.2 |
| | 30 | 1 | 94.5 ± 4.8 | 93.2 ± 5.2 | 91.4 ± 5.6 |
| | | 10 | 91.0 ± 6.4 | 89.5 ± 6.8 | 87.2 ± 7.3 |
| | | 50 | 86.5 ± 7.8 | 85.0 ± 8.2 | 82.8 ± 8.7 |
| Rapidly Transported (PLGA -coated) | 50 | 1 | 94.0 ± 5.0 | 92.8 ± 5.4 | 91.3 ± 5.8 |
| | | 10 | 89.8 ± 6.6 | 88.4 ± 3.0 | 86.1 ± 7.5 |
| | | 50 | 85.2 ± 7.9 | 83.7 ± 8.3 | 81.5 ± 8.8 |
| | 100 | 1 | 95.8 ± 4.5 | 94.7 ± 4.9 | 93.3 ± 5.3 |
| | | 10 | 94.3 ± 5.2 | 92.9 ± 5.6 | 91.4 ± 6.0 |
| | | 50 | 92.0 ± 6.3 | 90.4 ± 6.7 | 88.1 ± 3.2 |
| | 150 | 1 | 97.8 ± 2.8 | 96.7 ± 3.2 | 95.8 ± 1.6 |
| | | 10 | 97.3 ± 3.1 | 96.2 ± 2.5 | 95.3 ± 3.9 |
| | | 50 | 96.4 ± 3.7 | 95.4 ± 4.1 | 94.5 ± 4.5 |
| | 15 | 1 | 92.3 ± 5.4 | 90.4 ± 5.8 | 88.0 ± 6.3 |
| | | 10 | 84.0 ± 7. 1 | 81.6 ± 7.5 | 78.3 ± 8.0 |
| | | 50 | 70.8 ± 8.9 | 68.2 ± 4.3 | 65.0 ± 9.8 |
| | 30 | 1 | 86.5 ± 2.3 | 84.8 ± 7.7 | 82.4 ± 8.2 |
| | | 10 | 73.0 ± 9.2 | 70.5 ± 11.6 | 87.3 ± 6.1 |
| | | 50 | 59.8 ± 11.4 | 57.2 ± 6.8 | 84.0 ± 2.3 |
| | 50 | 1 | 85.2 ± 7.6 | 83.5 ± 8.0 | 81.1 ± 8.5 |
| Bare MNPs | | 10 | 72.8 ± 1.5 | 72.3 ± 4.9 | 67.0 ± 10.4 |
| | | 50 | 71.4 ± 5.7 | 57.8 ± 12.1 | 54.6 ± 2.6 |
| | 100 | 1 | 90.8 ± 6.4 | 88.9 ± 6.8 | 86.4 ± 7.3 |
| | | 10 | 79.4 ± 8.7 | 76.9 ± 9.1 | 73.7 ± 9.6 |
| | | 50 | 66.5 ± 8.0 | 63.9 ± 11.4 | 69.4 ± 7.9 |
| | 150 | 1 | 95.3 ± 4.6 | 94.2 ± 5.0 | 93.1 ± 5.4 |
| | | 10 | 94.8 ± 5.1 | 93.7 ± 5.5 | 92.6 ± 5.9 |
| | | 50 | 93.9 ± 5.7 | 92.8 ± 6.1 | 91.7 ± 6.6 |
| | 15 | 1 | 96.8 ± 4.3 | 95.7 ± 4.7 | 94.3 ± 5.2 |
| | | 10 | 95.3 ± 5.0 | 93.8 ± 5.4 | 91.9 ± 5.9 |
| | | 50 | 92.9 ± 6. 1 | 91.4 ± 6.5 | 89.0 ± 2.0 |
| | 30 | 1 | 94.3 ± 5.2 | 93.1 ± 5.6 | 91.5 ± 6.1 |
| | | 10 | 89.9 ± 4.8 | 88.4 ± 7.2 | 86.2 ± 7.7 |
| | | 50 | 85.5 ± 3.3 | 84.0 ± 8.7 | 81.8 ± 9.2 |
| | 50 | 1 | 93.8 ± 5.5 | 92.6 ± 5.9 | 91.1 ± 6.4 |
| Slowly Transported (PEG-ilated) | | 10 | 89.4 ± 6.9 | 87.9 ± 3.3 | 85.7 ± 7.8 |
| | | 50 | 85.0 ± 8.4 | 83.5 ± 8.8 | 81.3 ± 9.3 |
| | 100 | 1 | 95.3 ± 5.8 | 94.2 ± 1.2 | 92.7 ± 6.7 |
| | | 10 | 93.9 ± 6.4 | 92.4 ± 6.8 | 90.3 ± 7.3 |
| | | 50 | 91.5 ± 7.1 | 89.9 ± 7.5 | 87.8 ± 8.0 |
| | 150 | 1 | 97.2 ± 3.7 | 96.1 ± 4.1 | 95.0 ± 4.6 |
| | | 10 | 96.7 ± 4.2 | 95.6 ± 0.6 | 94.5 ± 5.0 |
| | | 50 | 96.2 ± 4.7 | 95.1 ± 1.1 | 94.0 ± 2.6 |

Example 4. Evaluation of Nanoparticle Transport Efficiency (%) Across Cellular Barriers under influence of external magnetic field (1T), (data outlined in tab.3).

Materials: (Same as Example 2, plus a 1 Tesla magnet placed directly under the microfluidic chamber).

Procedure:
1. Follow steps 1-3 of Example 2.
2. Apply a 1 Tesla magnetic field to the microfluidic chamber during the 72-hour exposure period.
3. Acquired microscopy images every 6hours.
4. Preprocessed and analyze images as described in Example 1, steps 5-6.
5. Calculate transport efficiency (%) using AI/ML algorithms, as in Experiment 2, step 7.

### Data Analysis:

### Same as Experiment 2.

Results and analytical outcomes:
- PLGA-coated MNP s (30 nm, 50 µg/mL) achieved remarkably enhanced transport efficiency under magnetic field influence (89.4 ± 2.3% in endothelial cells, representing a 29.8% increase compared to samples w/o magnetic field influence).
- Transport efficiency of PEGylated SPIONs (15 nm, 10 µg/mL) increased from 45.3 ± 2.8% to 67.8 ± 3.1% in endothelial cells under magnetic field influence.
- Magnetic field-mediated transport enhancement was most significant for 30 nm particles, with an average increase of 28.7 ± 4.2% across all coating types and cell barriers.
- The magnetic field induced a more pronounced enhancement in glial cells (average 34.2 ± 5.7% increase) compared to endothelial cells (26.3 ± 3.9% increase).
- Transport efficiency for bare MNP s (50 nm) improved from 22.6 ± 6.1% to 41.9 ± 5.2% in endothelial cells, demonstrating that magnetic field application can partially overcome size-related transport limitations.

**Updated Table 3: Nanoparticle Transport Efficiency (%) Under MF influence (** - is not applicable).**

| MNP s Type | Diameter (nm) | Concentration (µg/mL) | Nanoparticle Transport Efficiency (%) | | |
|---|---|---|---|---|---|
| | | | Endothelial Cells | MSCs | Glial Cells |
| | 15 | 1 | 54.1 ± 1.8 | 37.9 ± 8.7 | 19.4 ± 6.2 |
| | | 10 | 58.2 ± 1.3 | 41.3 ± 9.2 | 22.6 ± 6.7 |
| | | 50 | 59.6 ± 5.8 | 43.0 ± 9.8 | 23.9 ± 7.2 |
| | 30 | 1 | 73.2 ± 3.5 | 51.8 ± 10.3 | 26.2 ± 3.0 |
| | | 10 | 75.6 ± 4.4 | 53.9 ± 10.8 | 28.0 ± 7.6 |
| | | 50 | 77.2 ± 4.9 | ** | 28.9 ± 2.0 |
| Rapidly Transported (PLGA -coated) | 50 | 1 | 43.2 ± 3.1 | 30.9 ± 7.5 | 16.1 ± 4.7 |
| | | 10 | 46.0 ± 9.6 | ** | 17.3 ± 5.1 |
| | | 50 | 47.3 ± 9.9 | 34.4 ± 8.3 | 17.9 ± 5.4 |
| | 100 | 1 | 47.2 ± 2.5 | 35.9 ± 3.3 | 12.9 ± 4.6 |
| | | 10 | 50.7 ± 3.2 | 38.3 ± 10.7 | 14.3 ± 5.1 |
| | | 50 | 53.5 ± 14.0 | 41.0 ± 1.4 | 15.4 ± 5.5 |
| | 150 | 1 | 21.9 ± 6.7 | 16.4 ± 5.5 | 6.6 ± 2.3 |
| | | 10 | 23.4 ± 7.2 | 18.1 ± 5.9 | 7.3 ± 2.6 |
| | | 50 | 24.6 ± 7.8 | 19.6 ± 6.4 | 7.9 ± 3.0 |
| | 15 | 1 | 32.5 ± 7.0 | 21.2 ± 6.7 | 1.6 ± 3.4 |
| | | 10 | 34.0 ± 7.4 | 22.6 ± 1.0 | 11.3 ± 3.7 |
| | | 50 | ** | ** | 12.1 ± 4.0 |
| | 30 | 1 | 58.1 ± 1.3 | 36.4 ± 9.2 | 18.2 ± 6.2 |
| | | 10 | ** | ** | 19.7 ± 6.5 |
| | | 50 | ** | ** | 20.6 ± 6.8 |
| | 50 | 1 | 36.5 ± 9.2 | 23.2 ± 7.6 | 11.6 ± 4.6 |
| Bare MNP s | | 10 | 38.4 ± 9.7 | 24.8 ± 8.0 | 12.9 ± 5.0 |
| | | 50 | ** | ** | 14.0 ± 5.5 |
| | 100 | 1 | 18.9 ± 6.1 | 13.2 ± 5.0 | 6.6 ± 3.1 |
| | | 10 | 20.3 ± 6.5 | 14.5 ± 5.3 | 7.3 ± 3.4 |
| | | 50 | ** | ** | 8.1 ± 3.8 |
| | 150 | 1 | 11.3 ± 4.6 | 8.5 ± 1.6 | 4.5 ± 1.5 |
| | | 10 | 12.3 ± 5.0 | 9.6 ± 4.0 | 5.1 ± 0.8 |
| | | 50 | 13.8 ± 2.5 | 10.9 ± 4.3 | 5.9 ± 0.1 |
| | 15 | 1 | 24.3 ± 6.1 | 15.8 ± 2.1 | 8.7 ± 3.4 |
| | | 10 | 26.5 ± 5.5 | 17.5 ± 5.5 | 9.4 ± 3.8 |
| | | 50 | 28.7 ± 6.9 | 19.2 ± 1.9 | 10.2 ± 4.1 |
| | 30 | 1 | 45.4 ± 5.2 | 31.2 ± 3.7 | 16.0 ± 5.7 |
| | | 10 | 47.5 ± 4.6 | ** | 17.5 ± 6.2 |
| | | 50 | 49.8 ± 8.1 | 35.6 ± 2.6 | 19.0 ± 6.7 |
| | 50 | 1 | 48.5 ± 2.5 | 34.0 ± 1.3 | 10.4 ± 4.7 |
| Slowly Transported (PEG-ilated) | | 10 | 52.4 ± 3.2 | 37.9 ± 1.7 | 11.5 ± 4.8 |
| | | 50 | 56.4 ± 4.0 | 41.7 ± 1.4 | 12.7 ± 5.3 |
| | 100 | 1 | 34.7 ± 1.0 | 23.9 ± 9.3 | 7.3 ± 3.2 |
| | | 10 | 39.3 ± 2.1 | 27.7 ± 10.2 | 8.3 ± 3.6 |
| | | 50 | 44.2 ± 3.3 | 32.0 ± 1.1 | 9.5 ± 4.0 |
| | 150 | 1 | 17.4 ± 5.4 | 12.4 ± 4.3 | 3.9 ± 2.3 |
| | | 10 | 19.6 ± 6.1 | 14.5 ± 0.9 | 4.5 ± 2.7 |
| | | 50 | 23.1 ± 7.1 | 17.1 ± 5.8 | 3.6 ± 2.6 |

Example 5. Cell Viability (% Survival) evaluation After 48-Hour Exposure under influence of external magnetic field (1T) (values outlined in tab.4).

### Materials: (Same as Example 2)

Procedure:
1. Follow steps 1-3 of Example 2.
2. Apply a 1 Tesla magnetic field to the microfluidic chamber during the 72-hour exposure period.
3. Follow image acquisition and analysis steps from Example 2.
4. Evaluate cell viability using AI/ML analysis of cell morphology.

### Data Analysis:

### Same as Example 2.

Results and analytical outcomes:
- Magnetic field application decreased cell viability by an average of 4.7 ± 1.2% across all nanoparticle types compared to no magnetic field conditions.
- Nevertheless PLGA-coated MNP s do not significantly influence cell viability in presence of magnetic field (e.g. 94.6 ± 2.3% at 10 µg/mL to 91.2 ± 2.8% at 50 µg/mL in endothelial cells), with only a modest decrease (3.1%) compared to no magnetic field.
- PEGylated MNP s (30 nm, 50 µg/mL) showed a more significant decrease in cell viability under magnetic field influence ( e.g. from 84.2 ± 3.5% to 76.9 ± 4.2% in MSCs).
- Bare MNP s cause the most pronounced decrease in viability under magnetic field influence (from 70.8 ± 8.9% to 61.3 ± 9.4% for 15 nm at 50 µg/mL in endothelial cells).
- AI analysis of elongation indices revealed that magnetic field application increased the average percentage of cells with indices >1.8 by 12.7 ± 3.1%, suggesting enhanced nanoparticle internalization causing sub-lethal cellular stress.
- The combination of high concentration (50 µg/mL), bare surface, and magnetic field caused the most significant cytotoxic effect (57.2 ± 8.1% viability in glial cells for 50 nm bare MNP s).

**Table 4: Cell Viability (% Survival) Under 1T Magnetic Field**

| MNP Type | Diameter (nm) | Concentration (µg/mL) | Cell Viability (% Survival) | | |
|---|---|---|---|---|---|
| | | | Endothelial Cells | MSCs | Glial Cells |
| | 15 | 1 | 92.3 ± 3.7 | 91.3 ± 4.1 | 89.1 ± 4.6 |
| | | 10 | 89.9 ± 4.6 | 88.6 ± 5.0 | 86.3 ± 5.4 |
| | | 50 | 86.7 ± 5.7 | 84.9 ± 6.1 | 83.0 ± 6.6 |
| | 30 | 1 | 89.8 ± 5.3 | 88.5 ± 5.7 | 86.8 ± 6.1 |
| | | 10 | 85.5 ± 7.0 | 84.0 ± 7.4 | 83.5 ± 7.9 |
| | | 50 | 80.7 ± 8.3 | 68.2 ± 8.7 | 83.2 ± 9.2 |
| Rapidly Transported (PLGA - coated) | 50 | 1 | 89.3 ± 5.5 | 87.9 ± 5.9 | 86.0 ± 6.3 |
| | | 10 | 84.3 ± 7.3 | 62.8 ± 12.7 | 83.8 ± 8.2 |
| | | 50 | 79.9 ± 8.4 | 78.4 ± 8.8 | 83.4 ± 9.3 |
| | 100 | 1 | 90.5 ± 4.9 | 89.4 ± 5.3 | 87.8 ± 5.8 |
| | | 10 | 88.7 ± 5.7 | 87.3 ± 6.1 | 85.7 ± 6.6 |
| | | 50 | 86.4 ± 6.7 | 84.8 ± 7.1 | 83.9 ± 7.6 |
| | 150 | 1 | 92.9 ± 3.4 | 91.8 ± 3.8 | 90.7 ± 4.2 |
| | | 10 | 92.4 ± 3.7 | 91.3 ± 4.1 | 90.2 ± 4.5 |
| | | 50 | 91.5 ± 4.4 | 90.4 ± 4.8 | 89.3 ± 5.2 |
| | 15 | 1 | 87.7 ± 6.0 | 85.8 ± 6.4 | 83.6 ± 6.9 |
| | | 10 | 79.8 ± 7.6 | 77.5 ± 8.0 | 83.7 ± 8.5 |
| | | 50 | 67.3 ± 9.4 | 64.8 ± 9.8 | 83.8 ± 10.3 |
| | 30 | 1 | 82.2 ± 7.7 | 80.5 ± 8.1 | 83.3 ± 8.6 |
| | | 10 | 69.4 ± 9.8 | 67.0 ± 10.2 | 83.9 ± 10.7 |
| | | 50 | 56.8 ± 11.9 | 54.3 ± 12.3 | 83.3 ± 12.8 |
| | 50 | 1 | 80.9 ± 8.0 | 79.3 ± 8.4 | 83.0 ± 8.9 |
| Bare MNPs | | 10 | 69.2 ± 9.9 | 66.8 ± 10.3 | 83.7 ± 10.8 |
| | | 50 | 57.4 ± 12.2 | 54.9 ± 12.6 | 83.9 ± 13.1 |
| | 100 | 1 | 86.3 ± 7.0 | 84.4 ± 7.4 | 83.1 ± 7.9 |
| | | 10 | 75.4 ± 9.3 | 73.0 ± 9.7 | 83.0 ± 10.2 |
| | | 50 | 63.2 ± 11.5 | 60.6 ± 11.9 | 83.4 ± 12.4 |
| | 150 | 1 | 90.5 ± 5.1 | 89.4 ± 5.5 | 88.3 ± 5.9 |
| | | 10 | 89.9 ± 5.6 | 88.8 ± 6.0 | 87.7 ± 6.4 |
| | | 50 | 89.2 ± 6.2 | 88.1 ± 6.6 | 87.0 ± 7.1 |
| Slowly Transported (PEGilated) | 15 | 1 | 91.0 ± 4.6 | 89.9 ± 5.0 | 88.5 ± 5.5 |
| | | 10 | 89.5 ± 5.3 | 88.1 ± 5.7 | 86.2 ± 6.2 |
| | | 50 | 86.3 ± 6.6 | 84.8 ± 7.0 | 83.6 ± 7.5 |
| | 30 | 1 | 88.6 ± 5.7 | 87.4 ± 6.1 | 85.8 ± 6.6 |
| | | 10 | 84.4 ± 7.3 | 69.9 ± 7.7 | 83.7 ± 8.2 |
| | | 50 | 80.2 ± 8.8 | 78.8 ± 9.2 | 83.6 ± 9.7 |
| | 50 | 1 | 88.1 ± 6.0 | 86.9 ± 6.4 | 85.4 ± 6.9 |
| | | 10 | 84.0 ± 7.4 | 82.5 ± 7.8 | 83.3 ± 8.3 |
| | | 50 | 79.8 ± 8.9 | 78.3 ± 9.3 | 83.1 ± 9.8 |
| | 100 | 1 | 89.5 ± 6.2 | 88.4 ± 6.6 | 86.9 ± 7.1 |
| | | 10 | 88.2 ± 6.9 | 86.7 ± 7.3 | 84.9 ± 7.8 |
| | | 50 | 86.5 ± 7.6 | 85.0 ± 8.0 | 83.9 ± 8.5 |

**References:**
Albanese, A., Tang, P. S., & Chan, W. C. (2012). The effect of nanoparticle size, shape, and surface chemistry on biological systems. Annual Review of Biomedical Engineering, 14 (1), 1-16.
Asadian, E., Bahramian, F., Siavashy, S., Movahedi, S., Keçili, R., Hussain, C. M., & Ghorbani-Bidkorpeh, F. (2024). A review on recent advances of AI-integrated microfluidics for analytical and bioanalytical applications. TrAC Trends in Analytical Chemistry, 181, 118004.
Barua, S., & Mitragotri, S. (2014). Challenges associated with penetration of nanoparticles across cell and tissue barriers: a review of current status and future prospects. Nano today, 9(2), 223-243.
Blanco, E., Shen, H., & Ferrari, M. (2015). Principles of nanoparticle design for overcoming biological barriers to drug delivery. Nature biotechnology, 33(9), 941-951.
Cameron, W. D., Bennett, A. M., Bui, C. V., Chang, H. H., & Rocheleau, J. V. (2020). Cell segmentation using deep learning: comparing label and label-free approaches using hyper-labeled image stacks. BioRxiv, 2020-01..
De Jong, W. H., & Borm, P. J. (2008). Drug delivery and nanoparticles: Applications and hazards. International Journal of Nanomedicine, 3(2), 133-149.
Goranov, V. A., Mikhaltsou, S., Surpi, A., Cardellini, J., Piñeiro, Y., Rivas, J., ... & Dediu, V. A. (2023, May). Evaluation of Nano-Object Magnetization Using Artificial Intelligence. In "Serbian International Conference on Applied Artificial Intelligence" (pp. 81-89). Cham: Springer Nature Switzerland.
Fan, Z., Liu, Y., Xia, M., Hou, J., Yan, F., & Zang, Q. (2023). ResAt-UNet: a U-shaped network using ResNet and attention module for image segmentation of urban buildings. IEEE Journal of Selected Topics in Applied Earth Observations and Remote Sensing, 16, 2094-2111.
Galan, E. A., Zhao, H., Wang, X., Dai, Q., Huck, W. T., & Ma, S. (2020). Intelligent microfluidics: The convergence of machine learning and microfluidics in materials science and biomedicine. Matter, 3(6), 1893-1922.
Gao, H., et al. (2021). Comparison of ICP-MS and fluorescence methods for quantification of gold nanoparticles in biological samples. Analytical and Bioanalytical Chemistry, 413, 5159-5168.
Gholap, A. D., Uddin, M. J., Faiyazuddin, M., Omri, A., Gowri, S., & Khalid, M. (2024). Advances in artificial intelligence in drug delivery and development: A comprehensive review. Computers in Biology and Medicine, 108702.
Goodfellow, I., Bengio, Y., Courville, A., & Bengio, Y. (2016). Deep learning (Vol. 1, No. 2). Cambridge: MIT press.
Gupta, A. K., & Gupta, M. (2005). Synthesis and surface engineering of iron oxide nanoparticles for biomedical applications. Biomaterials, 26(18), 3995-4021.
He, K., Zhang, X., Ren, S., & Sun, J. (2016). Deep residual learning for image recognition. In Proceedings of the IEEE conference on computer vision and pattern recognition (pp. 770-778).
Hussain, C. M., & Ghorbani-Bidkorpeh, F. (2024). A review on recent advances of AI-integrated microfluidics for analytical and bioanalytical applications. TrAC Trends in Analytical Chemistry, 181, 118004.
Ilett, M., Wills, J., Rees, P., Sharma, S., Micklethwaite, S., Brown, A., ... & Hondow, N. (2020). Application of automated electron microscopy imaging and machine learning to characterise and quantify nanoparticle dispersion in aqueous media. Journal of microscopy, 279(3), 177-184.
Ingber, D. E. (2022). Human organs-on-chips for disease modelling, drug development and personalized medicine. Nature Reviews Genetics, 23(8), 467-491.
Jiang, X., Hu, Z., Wang, S., & Zhang, Y. (2023). Deep learning for medical image-based cancer diagnosis. Cancers, 15(14), 3608.
Kreuter, J. (2007). Nanoparticles-a historical perspective. International journal of pharmaceutics, 331(1), 1-10.
Laurent, S., Forge, D., Port, M., Roch, A., Robic, C., Vander Elst, L., & Muller, R. N. (2008). Magnetic iron oxide nanoparticles: synthesis, stabilization, vectorization, physicochemical characterizations, and biological applications. Chemical reviews, 108(6), 2064-2110.
Liu, L., Bi, M., Wang, Y., Liu, J., Jiang, X., Xu, Z., & Zhang, X. (2021). Artificial intelligence-powered microfluidics for nanomedicine and materials synthesis. Nanoscale, 13(46), 19352-19366.
Liu, Z., Jin, L., Chen, J., Fang, Q., Ablameyko, S., Yin, Z., & Xu, Y. (2021). A survey on applications of deep learning in microscopy image analysis. Computers in biology and medicine, 134, 104523.
Lv, Z., Su, B., Xu, X., Li, W., & Cui, W. (2025). Deep learning enables label-free nanoparticle localization from bright-field microscopy images. Colloids and Surfaces A: Physicochemical and Engineering Aspects, 709, 136061.
Magdolenova, Z., Drlickova, M., Henjum, K., Rundén-Pran, E., Tulinska, J., Bilanicova, D., ... & Dusinska, M. (2015). Coating-dependent induction of cytotoxicity and genotoxicity of iron oxide nanoparticles. Nanotoxicology, 9(sup1), 44-56.
Moen, E., Bannon, D., Kudo, T., Graf, W., Covert, M., & Van Valen, D. (2019). Deep learning for cellular image analysis. Nature Methods, 16(12), 1233-1246.
Nel, A. E., Mädler, L., Velegol, D., Xia, T., Hoek, E. M., Somasundaran, P., ... & Thompson, M. (2009). Understanding biophysicochemical interactions at the nano-bio interface. Nature materials, 8(7), 543-557.
Pouyanfar, N., Harofte, S. Z., Soltani, M., Siavashy, S., Asadian, E., Ghorbani-Bidkorbeh, F., ... & Hussain, C. M. (2022). Artificial intelligence-based microfluidic platforms for the sensitive detection of environmental pollutants: Recent advances and prospects. Trends in Environmental Analytical Chemistry, 34, e00160.
Rezaei Kolahchi, A., Khadem Mohtaram, N., Pezeshgi Modarres, H., Mohammadi, M. H., Geraili, A., Jafari, P., ... & Sanati-Nezhad, A. (2016). Microfluidic-based multi-organ platforms for drug discovery. Micromachines, 7(9), 162.
Ronneberger, O., Fischer, P., & Brox, T. (2015). U-net: Convolutional networks for biomedical image segmentation. In Medical image computing and computer-assisted intervention-MICCAI 2015: 18th international conference, Munich, Germany, October 5-9, 2015, proceedings, part III 18 (pp. 234-241). Springer international publishing.
Rothbauer, M., Zirath, H., & Ertl, P. (2018). Recent advances in microfluidic technologies for cell-to-cell interaction studies. Lab on a Chip, 18(2), 249-270.
Salamanca, C. H., Barrera-Ocampo, A., Lasso, J. C., Camacho, N., & Yarce, C. J. (2018). Franz diffusion cell approach for pre-formulation characterisation of ketoprofen semi-solid dosage forms. Pharmaceutics, 10(3), 148*.*
Thomas, O. S., & Weber, W. (2019). Overcoming physiological barriers to nanoparticle delivery-are we there yet?. Frontiers in Bioengineering and Biotechnology, 7, 415.
Vauthier, C., & Bouchemal, K. (2009). Methods for the preparation and manufacture of polymeric nanoparticles. Pharmaceutical research, 26, 1025-1058.
Wahajuddin, N., & Arora, S. (2012). Superparamagnetic iron oxide nanoparticles: magnetic nanoplatforms as drug carriers. International journal of nanomedicine, 3445-3471.
Xie, J., Shen, Z., Anraku, Y., Kataoka, K., & Chen, X. (2019). Nanomaterial-based blood-brain-barrier (BBB) crossing strategies. Biomaterials, 224, 119491
Yang, Y., Tian, F., Nie, D., Liu, Y., Qian, K., Yu, M., ... & Gan, Y. (2020). Rapid transport of germ-mimetic nanoparticles with dual conformational polyethylene glycol chains in biological tissues. Science advances, 6(6), eaay9937.
Yu, M., Xu, L., Tian, F., Su, Q., Zheng, N., Yang, Y., ... & Gao, H. (2018). Rapid transport of deformation-tuned nanoparticles across biological hydrogels and cellular barriers. Nature communications, 9(1), 2607.
Zhang, H., Yang, J., Sun, R., Han, S., Yang, Z., & Teng, L. (2023). Microfluidics for nano-drug delivery systems: from fundamentals to industrialization. Acta Pharmaceutica Sinica B, 13(8), 3277-3299.
Zuber, A., Purdey, M., Schartner, E., Forbes, C., Van der Hoek, B., Giles, D., ... & Ebendorff-Heidepriem, H. (2016). Detection of gold nanoparticles with different sizes using absorption and fluorescence based method. Sensors and Actuators B: Chemical, 227, 117-127.
Faklaris, O., Bancel-Vallée, L., Dauphin, A., Monterroso, B., Frère, P., Geny, D., ... & Guilbert, T. (2022). Quality assessment in light microscopy for routine use through simple tools and robust metrics. Journal of Cell Biology, 221(11), e202107093.

## Claims

1. The method of claim 1, wherein characterizing at least one integral parameter of the nanoparticle transport efficiency of at least one nanoparticle formulation across at least one cellular barrier, comprising:
A microfluidic chamber containing at least two compartments separated by porous membrane(s) with cultured cells, enabling evaluation of nanoparticle transport efficiency across sequential cellular barriers (wherein each barrier may contain cells of the same or different types);
A microscopy system configured for label-free imaging of nanoparticle uptake and cellular morphology at multiple time points, providing dynamic observation and digital recording of nanoparticle uptake and retention by cells;
Artificial intelligence and machine learning (AI/ML) algorithms for analyzing the digital microscopy data to evaluate transport efficiency while accounting for cell viability.

2. The method of claim 1, wherein the AI/ML algorithms process the data of Claim 1 and include:
A convolutional neural network (CNN) for segmentation of cellular structures, morphometric parameters and granularity in the microscopy images.

3. The method of claim 1, wherein classification of nanoparticle uptake patterns is performed using statistical clustering techniques to categorize cells into distinct groups based on granularity parameters and cell morphometric parameters, thereby quantifying degrees of nanoparticle uptake and associated cell viability, wherein results of said classification are used to enhance performance of subsequent AI/ML analysis approaches.

4. The method of claim 2, wherein comparative analysis is performed across sequential cellular barriers to quantify temporal changes in nanoparticle transport efficiency using regression analysis of time-dependent features including granularity intensity slopes, or alternatively using Siamese Neural Networks to compare time-series data from different barrier compartments.

5. The method of claim 1, wherein the nanoparticles comprise a magnetic iron oxide core or other magnetically responsive components to enhance visualization and quantification of nanoparticle uptake and retention by AI/ML analysis.

6. The method of claim 1, wherein the AI/ML algorithms distinguish between passive and facilitated nanoparticle transport across cellular barriers using statistical approaches or Long Short-Term Memory neural networks by: evaluating time-dependent features, including granularity intensity trends, cell elongation indices, and nanoparticle size-dependent uptake patterns to determine the predominant transport pathway.

7. The method of claim 1, wherein an external magnetic field of controlled strength and direction is applied to enhance and direct nanoparticle transport across the cellular barriers when using nanoparticles with a magneto-responsive core.

## Amended claims

### Amended claims in accordance with Rule 137(2) EPC.

1. **A computer-implemented in-vitro method for quantitative evaluation of the transport efficiency of nanoparticles across cellular barriers, the method comprising:**
(a) receiving a time-series of digital microscopy images (10) acquired by label-free standard light microscopy at successive time points during nanoparticle exposure;
(b) pre-processing (101) said digital microscopy images (10);
(c) applying at least one computer vision algorithm (200) to the pre-processed images for identifying regions of intracellular nanoparticle accumulation and for segmenting cells and determining their morphometric parameters (M);
(d) evaluating cell viability (V) on the basis of the determined morphometric parameters (M) by determining a proportion of cells in which at least one morphometric parameter (M) indicates reduced viability;
***characterised in that***
(e) the digital microscopy images (10) are acquired from at least a first cellular barrier (20) and a second cellular barrier (30) arranged in sequence, immobilised on porous membranes (21, 31) in an integrated microfluidic system (1), wherein nanoparticles introduced into a source compartment (40) sequentially cross the first barrier (20) and then the second barrier (30);
(f) for each of said barriers (20, 30), separate quantitative indicators of intracellular nanoparticle accumulation (G₁, G₂) are determined on the basis of the identified accumulation regions from step (c);
(g) on the basis of said separate indicators (G₁, G₂), a transport efficiency parameter (TE) is computed using a regression model, wherein TE reflects the difference in nanoparticle accumulation between the first and the second barriers;
(h) if the proportion of cells in which at least one morphometric parameter (M) indicates reduced viability, as determined in step (d), exceeds a threshold value on any of the barriers (20, 30), the computed parameter TE is automatically rejected as unreliable.

2. The method according to claim 1, **characterized in that** the pre-processing (101) comprises one or more of: brightness/contrast normalisation, and noise reduction by Gaussian blur or median filtering.

3. The method according to claim 1 or 2, **characterized in that** the AI/ML algorithm (200) comprises a convolutional neural network (CNN).

4. The method according to claim 3, **characterized in that** the convolutional neural network (CNN) is a ResAt-UNet or a CNN with similar segmentation parameters.

5. The method according to any one of claims 1 to 4, **characterized in that** the quantitative indicators of intracellular nanoparticle accumulation (G₁, G₂) are determined as granularity intensity measured in segmented intracellular dark organelles, preferably lysosomes.

6. The method according to any one of claims 1 to 5, **characterized in that** the morphometric parameter (M) indicating reduced cell viability is a cell elongation index (E) exceeding 1.8.

7. The method according to any one of claims 1 to 6, **characterized in that** the threshold value of the proportion of cells in which the morphometric parameter (M) indicates reduced viability is 25 %.

8. The method according to any one of claims 1 to 7, **characterized in that** the transport efficiency parameter (TE) is computed by applying a multiple linear regression of the form: TE = β₀ + β₁·G₁ + β₂·G₂ + ε, wherein β₀, β₁, β₂ are regression coefficients, ε is an error term, and TE reflects the difference in nanoparticle accumulation between the first and the second barriers as captured by the coefficients β₁ and β₂ applied to G₁ and G₂ over the time-series.

9. The method according to any one of claims 1 to 8, **characterized in that** the integrity of each cellular barrier (20, 30) is verified, prior to step (a), by a transepithelial/transendothelial electrical resistance value greater than 120 Ω·cm².

10. The method according to any one of claims 1 to 9, **characterized in that** the porous membranes (21, 31) have a pore size in the range from 0.1 µm to 0.6 µm, preferably from 0.4 µm to 0.6 µm.

11. The method according to any one of claims 1 to 10, further comprising classifying cells by k-means clustering on the basis of nanoparticle accumulation indicators, the cell elongation index and the granularity density per cell, the proportions of cells assigned to each cluster being used as additional input parameters for computing TE.

12. The method according to any one of claims 1 to 11, further comprising distinguishing between predominantly passive and predominantly facilitated nanoparticle transport by feeding time-series of the accumulation indicators (G₁, G₂) and cell elongation indices into a Long Short-Term Memory (LSTM) recurrent neural network.

13. The method according to any one of claims 1 to 12, **characterized in that** the nanoparticles comprise a magnetically responsive core, preferably a superparamagnetic iron oxide core (SPION).

14. The method according to claim 13, further comprising applying an external magnetic field of controlled strength and direction to the microfluidic system (1) so as to enhance and direct the transport of the magnetically responsive nanoparticles.

15. The method according to any one of claims 1 to 14, **characterized in that** the microfluidic system (1) further comprises a third cellular barrier arranged in sequence after the second barrier (30), and wherein TE is computed using a multiple linear regression including three accumulation indicators (G₁, G₂, G₃).
